# EUROPEAN PATENT APPLICATION

(11) **EP 3 813 076 A1**
(43) Date of publication of application: **28.04.2021**
(21) Application number: 20150625.0
(22) Date of filing: 07.01.2020
(51) Int. Cl.: G16H 40/20, G16H 50/20, G16H 10/60

(54) **APPARATUS AND METHOD FOR CONTROLLING A SYSTEM OF RESOURCES**

(30) Priority: 24.10.2019 US 201962925262 P
(71) Applicant: Koninklijke Philips N.V., 5656 AG Eindhoven (NL)
(72) Inventor: HENDRIKS, Cornelis Petrus, 5656 AE Eindhoven (NL); BULUT, Murtaza, 5656 AE Eindhoven (NL); COX, Lieke Gertruda Elisabeth, 5656 AE Eindhoven (NL); LAVEZZO, Valentina, 5656 AE Eindhoven (NL)
(74) Representative: Steenbeek, Leonardus Johannes

(57) **Abstract**

An approach is proposed for managing human operators (22), the approach comprising managing power delivery to sensor components (24) carried by the human operators based on a need for the human operators for performing a task. The human operators may be clinical staff in a hospital, and carry sensors for monitoring location and/or physiological parameters of the staff. The sensors (24) are activated and deactivated based on resource request signals received indicative of need for a particular kind of human assistance.

## Description

### FIELD OF THE INVENTION

This invention relates to an apparatus and method for controlling a system of resources, and in particular to controlling power supply of sensors of the system.

### BACKGROUND OF THE INVENTION

A recent development in technology is the so-called digital twin (DT) concept. In this concept, a digital representation (the digital twin) of a physical system is provided and connected to its physical counterpart, for example through the Internet of things as explained in US 2017/286572 A1. Through this connection, the digital twin typically receives data pertaining to the state of the physical system, such as sensor readings or the like, based on which the digital twin can predict the actual or future status of the physical system, e.g. through simulation, as well as analyze or interpret a status history of the physical twin. In case of electromechanical systems, this for example may be used to predict the end-of-life of components of the system, thereby reducing the risk of component failure as timely replacement of the component may be arranged based on its end-of-life as estimated by the digital twin.

Digital twins can be used for a range of applications to model and simulate the state of any range of physical systems. They may be used within factory environments for example to model in real time the state of one or more machines such as conveyor belts, processing units, or assembly robots for instance.

Digital twin technology is also becoming of interest in the medical field, as it provides an approach to more efficient medical care provision. For example, the digital twin may be built using imaging data of the patient, e.g. a patient suffering from a diagnosed medical condition as captured in the imaging data, as for instance is explained by Dr Vanessa Diaz in https://www.wareable.com/health-and-wellbeing/doctor-virtual-twin-digital-patient-ucl-887 as retrieved from the Internet on 29 October 2018. Such a digital twin may serve a number of purposes. Firstly, the digital twin rather than the patient may be subjected to a number of virtual tests, e.g. treatment plans, to determine which treatment plan is most likely to be successful to the patient. This therefore reduces the number of tests that physically need to be performed on the actual patient.

The digital twin of the patient may be used to predict the onset, treatment or development of such medical conditions of the patient using a patient-derived digital model, e.g. a digital model that has been derived from medical image data of the patient. In this manner, the medical status of a patient may be monitored without the routine involvement of a medical practitioner, e.g. thus avoiding periodic routine physical checks of the patient. Instead, only when the digital twin predicts a medical status of the patient indicative of the patient requiring medical attention based on the received sensor readings may the digital twin arrange for an appointment to see a medical practitioner to be made for the patient. This typically leads to an improvement in the medical care of the patient, as the onset of certain diseases or medical conditions may be predicted with the digital twin, such that the patient can be treated accordingly at an early stage, which not only is beneficial to the patient but can also reduce (treatment) costs. Moreover, major medical incidents that the patient may be about to suffer may be predicted by the digital twin based on the monitoring of the patient's sensor readings, thereby reducing the risk of such incidents actually occurring. Such prevention avoids the need for the provision of substantial aftercare following such a major medical incident, which also alleviates the pressure on a healthcare system otherwise providing such aftercare.

Digital twins (DTs) can also be developed for clinical staff or other human operators within the clinical environment. These can model for example parameters of the operators relevant to their competency or usefulness for assisting in medical actions. This can include their clinical expertise, their clinical specialism, their level of experience, and more immediate factors such as fatigue level, stress level, amount of time on duty since a rest break, alertness and other factors.

Production facilities and hospitals might thus make use of large systems of multiple DTs for both staff or operators, and patients or machines, which can provide more intelligent insight into demand for, and availability of, various resources. Such systems however naturally entail very large volumes of data traffic and high computational demands which may make them prohibitively complex or expensive to implement and maintain.

### SUMMARY OF THE INVENTION

Developments are therefore needed to reduce overall complexity of such systems and reduce computational and data traffic requirements. The invention is defined by the independent claims. The dependent claims define advantageous embodiments.

According to examples in accordance with an aspect of the invention, there is provided a control unit for interacting with a system of resources,
the system comprising a plurality of human operators, each of a particular human operator type, and each human operator carrying on their person a set of one or more sensors for use in monitoring at least one of: location of the operator and a physiological parameter of the operator, and
the control unit communicatively coupleable in use with the sensor sets of the plurality of human operators; and
the control unit configured for receiving request signals indicative of a need for a particular human resource, the human resource associated with a particular one or more of the types of human operator; and
the control unit configured to control activation and deactivation of the sensors sets of the plurality of human operators dependent on received request signals.

There is thus provided a unit for managing the power and data collection of human object sensors in a system of human object resources. The system comprises a plurality of sensors which may for example be for collecting data for updating digital twins of the human operators, or for any other workforce monitoring purpose. The provided control unit dynamically controls activation and deactivation of sensors of the human operators based on information about current demand for human assistance. Essentially, the timing of sensor activation/deactivation is controlled based on the need for a human resource.

Thus rather than continuously collecting sensor data from human operators at all times, which naturally incurs high energy consumption, data traffic, and processing demand, sensors are kept by default powered down in an idle mode and only activated when there is a human resource demand that may call for the operator. At this point sensor data from the human operators can be collected for a short period, in order to allow an assessment to be made as to the optimal set of one or more operators to respond to the need for human intervention. For example, in the simplest case, a human operator closest to the patient or machine in need of assistance might be selected. Other factors however may also be taken into account such as expertise level and operator fatigue.

Energy demands and data storage requirements are thereby reduced since sensors are only powered on in instances when they are needed. This increases sensor battery life, reduces data flows, and reduces data processing and storage. Improvements are also provided in terms of data protection and user privacy, because data is only collected in instances when it is operationally necessary, reducing the collection and storage of unnecessary data about a person.

The control unit is associated with the system of resources. The control unit is for interacting (e.g. communicating) with one or more components of the system. For example, the system is at least for interacting with said sensors or sensor sets carried by human operators. As will be explained below, in further examples, the system includes further components, such as one or more digital twins, and the control unit may be for interacting (e.g. communicating) also with these digital twins. Further examples and explanations will be provided below.

The control unit may comprise in examples one or more processors. The steps of procedures implemented by the control unit may be implemented by a single processing component, or the processes maybe distributed among a plurality of different processing components, which may be incorporated in a single unit in one location or distributed among a plurality of units.

Activating and deactivating the sensors may include powering the sensors on and off. When a sensor is active, it may be powered on and collecting data. When a sensor is inactive, it is not collecting any data. When a sensor is inactive it may be powered off. In some examples, when a sensor is active, it may be in a (relative) high power mode, and when it is inactive, it may be in a lower power mode, for example an idle mode in which it is not collecting data. In the inactive mode it may nonetheless be communicable with the control unit for receiving control commands, e.g. a command triggering reactivation.

The control unit may be configured, responsive to each received request signal, to trigger activation of the sensor sets for each of the plurality of human operators of said particular one or more types. For example the control unit may issue one or more command signals for triggering activation of the sensor sets.

In advantageous examples, the system may further comprise a data storage unit, the data storage unit storing a personalized digital model for each of the human operators configured to model for the operator at least one or more of: location, agenda (e.g. future and past), stress level, time on duty since a rest break, fatigue level, alertness, and clinical experience.

These of course merely represent an exemplary subset of possible parameters which a digital twin of a human operator may model. A wide variety of different parameters may be modelled for the human operators to assist in the selection of an optimum one or more operators for a particular request for assistance. Further modelled parameters might include cumulative physical movement of the operator (i.e. indicative of physical exertion); computer usage data (e.g. text generated by the operator during their shift, which may be indicative of mental exertion), speech data (e.g. duration/energy of speech, e.g. measured by one or more acoustic transducers), and/or information about previous cases attended to during the same shift (e.g. severity of cases, number of patients).

In examples, the data storage unit may be a distributed system of multiple data storage units, or may consist of a single central data storage unit.

The sensors may be for collecting data suitable for updating said personalized digital models. For example the sensors may measure parameters which correspond to input parameters of the personalized digital models. They may correspond directly, or may collect data indicative of the one or more input parameters of the model.

The control unit may be configured, subsequent to activation of said sensor sets of said particular types of human operator, to collect data from each of the sensor sets, and to update each digital model of each human operator based on the data collected from the relevant operator's sensor set.

In advantageous embodiments, the control unit may be further configured to trigger deactivation of the sensor sets of the relevant one or more types of human operators subsequent to updating the digital twins.

The control unit may issue one or more command signals to the sensor sets for example, for triggering deactivation of the sensor sets.

The deactivation may be triggered immediately subsequent to the updating of the digital twins (i.e. as the next step afterwards) or it may be done after completion of one or more additional steps or processes, for example after selecting of a particular one or more of the human operators for responding to the human resource requirement, or after the requirement for the human resource has been fulfilled, for instance as indicated by acceptance of the assignment of human operators by a manager and/or by the selected one or more human operators themselves. This latter option will be explained in further detail in descriptions to follow.

In some examples, the control unit may be further configured to select one or more of the human operators of said particular one or more types based on data from the updated personal digital models of said human operators. For example, the control unit selects the optimal one or more of the human operators of the relevant operator types for responding to or attending the need for the human resource. By way of example, a physiological parameter of a patient may have moved outside of normal parameters, triggering generation of the request signal, indicative of a need for a clinician to attend the patient. The one or more types of human operator may include all doctors and all nurses. The control unit may gather sensor data from all doctors and all nurses indicative at least of a location of each doctor and each nurse and the closest one may then be selected as the optimal human operator for responding to the event. This represents however just one example, provided by way of illustration.

The selected most appropriate human operator in general may not simply be the person who is available at the nearest location; the determination may also take into account factors such as which operator is most rested, concentrated, focused, and/or knowledgeable for example.

The control unit may be configured, responsive to receipt of the request signal, to perform a step of determining the particular types of human operator associated with the human resource indicated by the request signal. In these examples, the control unit determines which one or more types of human operator are appropriate for responding to the particular human resource need. This may be determined for example using a simple lookup table, or may be determined using one or more algorithms, for example one or more machine learning algorithms.

The control unit may include a resource request module arranged to recurrently or continuously receive information indicative of a current state of one or more patients, to detect based on the information any need for a human resource, and to generate the one or more request signals for the control unit based on any such detection. The resource request module may be a separate component or its function may be performed by the same processing component(s) as the remainder of the functions of the control unit.

In these examples, the request signal or message is generated by the control unit itself responsive to patient information received for example from patient monitoring equipment. This information is monitored and any need for human intervention or attendance automatically detected, triggering the request signal.

In some examples, the resource request module may comprise, or may make use of, one or more digital twins. For example, there may be stored at a data storage unit a respective digital twin for each of a set of one or more patients being monitored. Information related to a state of each patient may be received at the control unit and provided to these digital twins to update each digital twin with information about the relevant patient. Each patient digital twin may then itself indicate any potential abnormality or problem with the patient indicative of a need for a particular human resource, or the control unit may probe or interrogate the digital twins recurrently to identify any problem which may indicate a need for a human resource. These represent examples only; further example will be described in detail later.

Thus, in these examples, the detecting the need for a human resource may be based on use of one or more personal digital models of the one or more patients.

Examples in accordance with a further aspect of the invention provide a method for interacting with a system of resources,
the system comprising
a plurality of human operators, each of a particular human operator type, and each human operator carrying on their person a set of one or more sensors for use in monitoring at least one of: location of the operator and a physiological parameter of the operator, and the method comprising
receiving request signals indicative of a need for a particular human resource, the human resource associated with a particular one or more of the types of human operator; and
controlling activation and deactivation of the sensors sets of the plurality of human operators dependent on received request signals.

In one or more embodiments, the method may comprise, responsive to receipt of the request signal, performing a step of determining the particular types of human operator associated with the human resource indicated by the request signal.

In accordance with one or more embodiments, the control unit may further comprise a data storage unit, the data storage unit storing a personalized digital model for each of the human operators configured to model for the operator at least one or more of: location, agenda (e.g. future and past), stress level, time on duty since a rest break, fatigue level, alertness, and clinical experience.

The method may further comprise, subsequent to activation of said sensor sets of said particular types of human operator, collecting data from each of the sensor sets, and updating each digital model of each human operator based on the data collected from said operator's sensor set.

In one or more embodiments, the method may further comprise triggering deactivation of said sensor sets of the one or more types of human operators subsequent to updating the digital twins.

In one or more embodiments, the method may further comprise selecting one or more of the human operators of said particular one or more types based on data from the updated personal digital models of said human operators.

Implementation options and details for each of the above steps and features may be understood and interpreted in accordance with the corresponding explanations and descriptions provided above for the apparatus aspect of the present invention (i.e. the control unit aspect).

Any of the examples, options or embodiment features or details described above in respect of the apparatus aspect of this invention (in respect of the control unit) may be applied or combined or incorporated into the method aspect of the invention.

Examples in accordance with a further aspect of the invention provide a computer program product comprising code unit configured, when run on a processor, the processor being communicatively coupled with sensor sets carried by a plurality of human operators, to cause the processor to perform a method in accordance with any example or embodiment described above or below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates in block diagram form an example control unit in accordance with one or more embodiments, and interaction of the control unit with a system of resources;
Fig. 2 outlines in block diagram form an example method in accordance with one or more embodiments; and
Fig. 3 outlines in block diagram form a further example method in accordance with one or more embodiments.

### DETAILED DESCRIPTION OF THE EMBODIMENTS

The invention will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an approach to managing components of a system of resources which includes human operators, and the approach comprising managing power delivery to sensor components carried by the operators based on predicted need for the human operators for performing a task. The human operators may be clinical staff in a hospital for example, and carry sensors for monitoring location and/or physiological parameters of the staff, and wherein the sensors are activated and deactivated based on resource request signals or messages received indicative of need for a particular kind of human assistance.

The method is for use as part of an apparatus or method of coordinating the system of human resources for responding to real-time human resource demands. The method is for reducing power consumption and data traffic in the system by intelligently managing when sensors are active and inactive based on real-time demand for human resources. Based on the resource request, sensors are activated for only of a subset of human operators who are capable of responding to the resource request. This avoids unnecessary power consumption, data transfer and data storage.

The sensors can be for monitoring the human operators, for collecting information relevant to the efficient organization and delivery of human resources to meet demand, for example based on which operators are closest to the patient or machine which needs attention and/or which operators are least fatigued or most experienced for the relevant task.

In an advantageous set of embodiments, the sensors can be for collecting data for updating personalized digital models (digital twins) for each of the human operators, which then reflect a real-time state of the human operators. Output parameters of the digital twins can then be probed or interrogated by a control unit for determining an optimal one or more human operators for responding to a need for assistance in a particular scenario. The output parameters may relate for instance to operator location, hours on duty since last break, operator fatigue, operator stress, operator specialism, operator heart rate and/or any other factors.

Patients or machines in the hospital or factory may also have digital twins created and associated with them for modelling a real-time state of the patient or machine.

These digital twins may monitor or predict the need for human interaction with the patient or machine in the near future. Thus the ongoing and upcoming demands for human resources, as well as the efficient delivery of them by human operators, can be assisted or mediated by use of digital twins.

Thus, production facilities and hospitals may evolve to a state in which digital twins of humans and devices or processes will interact to optimize or manage processes. For example, if a device (e.g. a conveyor belt, a robot or an imaging device) or a patient requires a human operation or intervention immediately or in the near future (e.g. by an operator, a nurse or a physician), for example as indicated by its digital twin, an optimal human operator may be selected based on output parameters of the population of human digital twins mirroring these operators.

To enable this, one option is the continuous monitoring of human operators with sensors in order to continuously update the status of their digital twins using the sensor data. However, to maintain such an abundance of connected human sensors continuously active and transmitting signals, and including the receiving, processing and storing of the collected data, imposes significant energy demands, and creates a high volume of data traffic. It furthermore necessitates large amounts of data storage space and processing resource. Furthermore, continuous tracking and data collection of humans may be non-compliant with data protection and privacy laws.

There is thus a need to minimize energy consumption and data collection, transfer and storage in monitored systems of human resources.

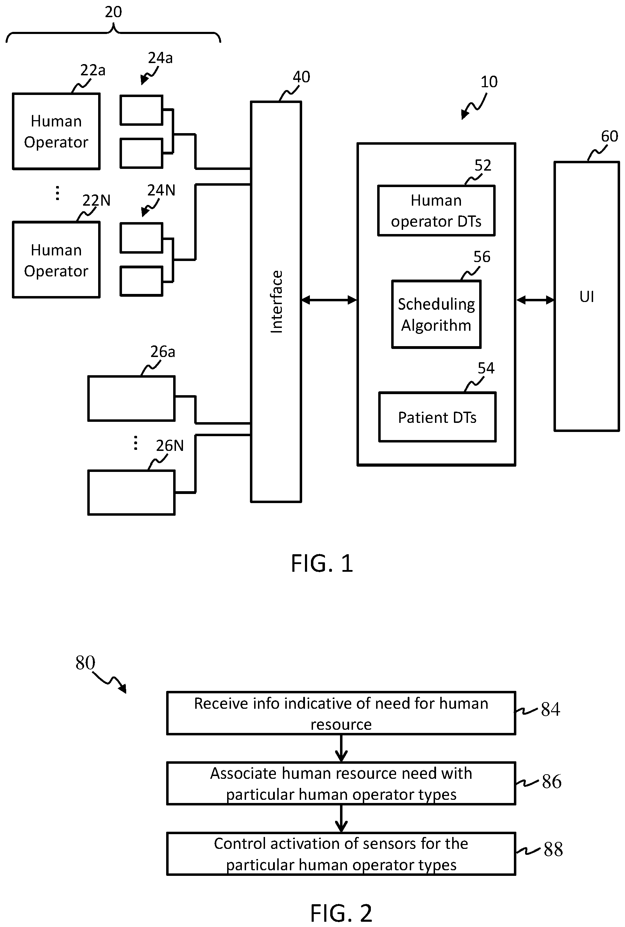

Fig. 1 schematically illustrates a control unit 10 in accordance with one or more embodiments of the invention. The control unit 10 is shown in conjunction with a system 20 of resources with which it is communicable and with which it interacts. The system of resources includes a plurality, N, of human operators 22. Each of the human operators is associated with a respective set of one or more sensors 24 for monitoring at least a position of the operator and optionally one or more physiological parameters of the human operator such as for example heart rate, stress level, blood oxygen level and/or any other parameters. Each human operator carries the respective set of sensors on his person. At least some of them may be wearable or body-mountable sensors for example.

Within the system 20, the human operators 22 are classified into different human operator types, for example based on their clinical role (e.g. nurse, doctor, surgeon, medical imaging operator, anesthetist and so on), and/or based on their clinical specialism, and/or based on their level or experience (e.g. junior doctor, senior doctor).

The control unit 10 is communicatively coupleable in use with the sensor sets 24a-24N of the plurality of human operators 22a-22N. The communicative coupling between the control unit 10 and the sensor sets 24 of the system 20 of resources may be mediated or implemented by an interface 40 which may comprise for example a wired or wireless connection, for example a wireless communication hub, or a wired network routing, or a wired or wireless internet connection, or any other suitable interface.

The control unit 10 is configured for receiving request or information signals indicative of a need for a particular human resource, the human resource associated with a particular one or more of the types of human operator.

The control unit 10 is further configured to control activation and deactivation of the sensors sets 24 of the plurality of human operators 22 dependent on received request signals.

For example, the control unit 10 may be configured, responsive to each received request signal, to trigger activation of the sensor sets 24 for each of the plurality of human operators 22 of said particular one or more types.

For example, the control unit 10 may be configured to deactivate the sensor sets 24 for each of the particular plurality of human operators of said particular one or more types after collection of data, for example after collection of a certain number of sensor readings or a certain quantity of data, or after a defined period of time, or after completion of one or more further pre-defined tasks or processes or steps.

In the example of Fig. 1, also communicatively coupled to the control unit 10 are a plurality of resource-consuming entities 26a-26N. In the example of a hospital application, these may be patients. In the example of a factory application, these may be machines or equipment or apparatus of the factory. For the purposes of the following description, these entities will be assumed to be patients in a hospital environment. However, it should be understood that this is only one example application and the functionality described may also be applied in analogous fashion to any other system comprising entities which may need intervention by human operators.

The plurality of patient 26 may be monitored by one or more patient sensors which monitor one or more patient parameters for each patient. These patient parameter data may be communicated to the control unit 10, for example via the interface 40, for use in determining or detecting need by any of the patients for a human resource. In some examples, the control unit includes a module arranged to receive and monitor this data and to detect any current or upcoming need for human resource based on the data, and to generate a request signal for the control unit based on this. In other examples, there may be a separate one or more units or modules, outside of the control unit, for receiving the patient data and detecting need for human resources by any of the patients and generating the request signals for communication to and receipt by the control unit 10.

The control unit 10 may comprise one or more processors. It may comprise a control unit or box containing one or more processors, or it may represent a distributed set of processing components, which may be distributed among a plurality of units.

In some examples, the control unit 10 may include a central processing module at which the request signals are received. They may be received from one or more other processing modules of the control unit, for example from a data storage unit storing one or more digital twins for patients, and/or a data storage unit storing one or more digital twins for the human operators, and/or a dedicated resource request module. A resource request module may be included for determining which particular human operator types may be appropriate for responding to a detected need for a human resource, e.g. a human intervention or operation. In other examples, such a module, or unit for implementing such functionality, may be provided outside of the control unit, and communicable with the control unit.

In some examples, the control unit 10 may be a cloud-based control unit, wherein the data storage functions are performed by cloud-based data storage, and processing functions are performed by cloud-based processing unit. The sensor sets 24 of the system 20 may communicate with the cloud based control unit via an internet connection.

As discussed above, in accordance with one or more advantageous embodiments, there may be included within the control unit 10 or the system 20 one or more personal digital models (digital twins) for each of the human operators. Data collected from the sensor sets 24 of the human operators may be used for updating these digital twins of the human operators. The human operator digital twins can be used by the control unit in performing a further function of determining an optimum one or more human operators for fulfilling the need for a human resource indicated by the request signal.

As also discussed above, in accordance with one or more advantageous embodiments, there may be included within the control unit 10 or the system 20 one more personal digital models (digital twins) for modelling each of the patients 26 (or other monitored entities). Patient data collected from the optional patient sensors may be used for updating these patient personal digital models. The patient digital models may be used by the control unit or another component or module in determining or detecting the need for human resource by any of the patients. For example, the patient digital twins are kept updated with patient data, and then output parameters of the twin model can be probed or interrogated to detect present or likely upcoming need for human intervention or assistance by the patient to address a clinical issue. This may trigger generation of a corresponding resource request signal, or it may trigger a resource request module to determine an appropriate one or more types of human operator for responding to the need, this module then generating the resource request signal.

Accordingly, in the particular example of Fig. 1, the control unit 10 further comprises a data storage unit, the data storage unit storing a personalized digital model 54 ("DT") for each of the human operators configured to model for the operator one or more of: location, agenda, fatigue level, experience. In other examples, the human operator digital models may be stored on data storage unit located outside of the control unit, for example as part of the system of resources.

As discussed, in this example, the human operator sensor sets 24 are for collecting data suitable for updating the personalized digital models 52 of the human operators 22.

In the example of Fig. 1, there is also included a data storage unit storing a personalized digital model 54 ("DT") for each of the patients. Although in Fig. 1, the patient digital models are shown stored in a different data storage unit to the human operator digital models, they may in other examples be stored in the same data storage unit.

By way of example the digital model 54 for each patient may encompass model parameters mapping onto one or more patient physiological or anatomical parameters, such as blood pressure, heart rate, blood oxygen level, medical image data showing tissue structures, breathing rate, breathing capacity and any other clinical parameters for the patient. Data collected from patient sensors associated with each of the patients 26 is used to update the patient digital twin models 54.

The control unit 10 is preferably further configured, subsequent to activation of said sensor sets 24 of said particular types of human operator, to collect data from each of the sensor sets, and to update each digital model 52 of each human operator 22 based on the data collected from the relevant operator's sensor set.

The control unit 10 is preferably further configured to trigger deactivation of said sensor sets 24 of the one or more types of human operators 22 subsequent to updating the digital twins 52 for those human operators.

Deactivation may be done immediately subsequent to the updating of the digital twins (i.e. as the next step afterwards) or it may be done after one or more further processes, for example after selecting a particular one or more of the human operators 22 for responding to the human resource requirement, or after the requirement for the human resource has been fulfilled, for instance as indicated by acceptance of the assignment of human operators by a manager and/or by the selected one or more human operators themselves. This latter option will be explained in further detail to follow.

In the example of Fig. 1, the control unit 10 further includes a scheduling algorithm 56 (for example a processing component configured to implement a scheduling algorithm). The scheduling algorithm may be configured for performing a function of coordinating or organizing the human operators, for example scheduling their use of time within the hospital or other environment.

The scheduling algorithm 56 may be configured at least to perform a function of selecting one or more of the human operators 22 for responding to the need for human resource indicated by the resource request signal. This is preferably done based on data from the updated personal digital models 52 of said human operators.

Although a scheduling algorithm 56 is included in the control unit 10 of Fig. 1 for performing the function of selecting the optimal human operators 22 for responding to the resource request, this function may instead be performed by any other component of the control unit 10, for example a further processing module or component in examples.

The selection made by the scheduling algorithm 56 is then output to a user interface 60 (UI) for communicating to a user or operator or manager or the system. For example, a manager having authority over co-ordination of the human operators may review the one or more human operators 22 selected by the scheduling algorithm via the UI 60. In some examples, the UI may permit a user to indicate whether or not they approve of the selection, and may allow the user to alter the selection.

As discussed, some embodiments make use of personalized digital models (digital twins) 52, 54 for the human operators 22 and/or for the patients 26 (or other monitored entities) of the system 20.

A digital twin 52 for a human operator typically provides a model of a plurality of relevant parameters of the human operator, i.e. the physical twin (relevant to the deployment of human operators for tasks). The digital twin may by way of example integrate artificial intelligence, machine learning and/or software analytics with spatial network graphs to create a 'living' digital simulation model of the relevant parameters of the human operator. The relevant parameters are parameters which allow selection of human operators for responding to human resource requests to be made. For example, the parameters modelled by each digital model (digital twin) may include one or more of: activity record of the operators, agenda of the operator (e.g. upcoming scheduled appointments), clinical (or other) role, clinical (or other) knowledge base, as well as current location of the operator, and preferably also one or more physiological parameters such as stress level, fatigue, concentration level.

These model output parameters may be calculated or determined by algorithms of the model based on various input parameters of the model, which can be updated recurrently using sensor data from the sets of human operator sensors 24. For example, the output parameters of fatigue level may be determined based on input parameter information such as

heart rate variability (see Saboul, D. and Hautier, C., 2019, "A New Algorithm to Reduce and Individualize HRV Recording Time", Journal of Medical Systems, (2019) 43:45),
actigraphs (see Zhu, Y. et al., 2017, "Wearable Sensors and Their Metrics for Measuring Comprehensive Occupational Fatigue: A Scoping Review", Proceedings of the Human Factors and Ergonomics Society 2017 Annual Meeting, pp. 1041-1045), and/or
shift schedules for the operator (Caldwell, J. et al., 2019, "Fatigue and its management in the workplace", Neuroscience and Biobehavioral Reviews Vol. 96, pp. 272-289 (section 11.13)).

Hence these input parameters of the model may be kept updated with real-time measured values for the human operators so that the model is continuously kept as a 'live' accurate replica of the state of the patient's anatomy. From the output parameters of the model, decisions as to an optimum selection of human operators for responding to a resource request signal may be determined.

In particular, at any given time, the current status of the human operator can be determined with the Digital Twin.

Furthermore, a predicted future status of the human operator and the effect of deployment of particular operators in a given circumstance can be determined. For example, using bio-mathematical models it is possible to predict the effect of (future) events such as scheduling factors and (chronic) sleep restrictions for human operators. See for example the paper: Caldwell, J. et al., 2019, "Fatigue and its management in the workplace", Neuroscience and Biobehavioral Reviews Vol. 96, pp. 272-289 (section 11.12).

As discussed above, personal digital models (digital twins) 54 of patients 26 (in the case of clinical environments) or machines or equipment (in the case of factory environments) may be used for detecting need for human intervention, calling for a human resource requirement.

By way of example, for patients in a hospital, the digital twin provides a model of a plurality of relevant physiological and/or anatomical parameters, wherein these output parameters of the model can be determined or calculated by model algorithms based on one or more model input parameters. The input parameters may be directly measurable parameters for the patient, such as one or more physiological parameters (e.g. heart rate, blood oxygen concentration, blood pressure etc.), and also imaging data of one or more portions of the anatomy of the patient.

In some examples, the patient digital twin may include a biophysical model of the patient. It may provide a model of both the elements and the dynamics of at least one or more portions of the anatomy of the patient (i.e. the physical twin). The digital twin may by way of example integrate artificial intelligence, machine learning and/or software analytics with spatial network graphs to create a 'living' digital simulation model of the at least portion of the patient's anatomy.

By way of non-limiting example, the patient may be admitted to a hospital for a cardiac-related illness. The at least portion of the patient's anatomy modelled by the patient digital twin may in this case be a part of the cardiac or arterial system of the patient, such that the digital twin comprises a model of this part of the anatomy of the patient. Such a living digital simulation may for example involve the use of a fluid dynamics model, a systemic model, a tissue deformation model and/or a fluid-structure interaction model in order to develop or update the digital twin based on adjustments of one or more input parameters of the model.

The personal digital model, i.e. the digital twin, of the patient may be initially developed from patient data, e.g. imaging data such as CT images, MRI images, and/or ultrasound images.

For example a medical scan may be conducted of the patient, and/or a set of one or more physiological or anatomical parameter measurements taken for the patient, and the digital model constructed based on this.

By way of example, a typical workflow for creating and validating a 3D, subject-specific biophysical model is depicted in "Current progress in patient-specific modeling", by Neal and Kerckhoff, 1, 2009, Vol. 2, pp. 111-126. For example, in case of a digital twin representing part of the cardiovascular system of the patient, such a biophysical model may be derived from one or more angiograms of the patient.

Development and implementation of digital twin models for various example applications are described in the literature for this field. By way of example, implementation details for various example digital twin models are described in the following papers: Gonzalez, D., Cueto, E. & Chinesta, F. Ann Biomed Eng (2016) 44: 35; Ritesh R. Rama & Sebastian Skatulla, Towards real-time cardiac mechanics modelling with patient-specific heart anatomies, Computer Methods in Applied Mechanics and Engineering (2018) 328; 47-74; Hoekstra, A, et al, Virtual physiological human 2016: translating the virtual physiological human to the clinic, interface Focus 8: 20170067; and "Current progress in patient-specific modeling", by Neal and Kerckhoff, 1, 2009, Vol. 2, pp. 111-126. Details are also outlined in "Computational Biomechanics for Medicine", Grand R. Joldes et al, Springer.

Sensor data from the patient may in some example be continuously or recurrently collected and used to update or develop the digital twin for the patient, so that the model is continuously kept as a 'live' accurate replica of the state of the patient's anatomy. The fundamentals of a patient-specific digital model for a given patient's anatomy may be developed in advance of deployment of the control unit or method of the invention, such that before the procedure begins, the digital model is an accurate representation of the current physical state of the portion of the anatomy of the patient of interest, and incorporates sufficient information and knowledge about the material properties and physical response characteristics to allow the model to be dynamically evolved or developed by adjusting model input parameters.

In general, the digital model, e.g. of an organ or tissue area of the patient, incorporates a number of different (e.g. heterogeneous) material properties as parameters of the model, which may include blood vessels, muscles, fat, lining tissue, bones, calcified areas, which each have specific (biomechanical) material properties. These material properties form parameters for the model which can be adjusted to thereby alter the physical state which is being simulated by the model. Often, these model parameters reflect measurable physiological or anatomical parameters of the patient or their anatomy. These parameters are kept updated with real-time measured values for the parameters of the patient so that the model is continuously kept as a 'live' accurate replica of the state of the patient's anatomy.

By probing or examining the updated or developed digital twin, the clinical state of the patient can be determined, and problems of pathologies that arise with the patient identified. The model furthermore may allow potential future problems for the patient to be predicted in advance based on developing the model along parameter trends that have been observed in the data and analyzing the resulting state of the patient. This can be used to pre-emptively address potential future medical problems before they arise. This may call for human intervention by one or more of the human operators for example.

Use of digital twins represents just one example implementation. In other examples, the sensor data may be fed to a control program or algorithm for processing and for determining an optimal one or more human operators. In this case, by way of example, the control unit may be configured to deactivate (e.g. switch off power to) the previously activated sensor sets after collection of sufficient data for performing said determination. By way of example, this may be after a defined number of sensor readings from the sensors, or a defined number of parameters, or after a defined amount of time, for example.

As discussed, in use, request signals are received at the control unit 10, or at least at a component or module of the control unit, indicative of a need for a particular human resource, the human resource associated with a particular one or more of the types of human operator.

In some examples, the control unit itself may be configured, responsive to receipt of the request signal, to perform a step of determining the particular types of human operator associated with the human resource indicated by the request signal. Alternatively, this information may be included in the request signal or message so that a separate step is not needed for determining this information.

In some examples, the control unit may include a resource request module arranged to recurrently or continuously receive information indicative of a current state of the one or more patients 26. The module may further be configured to detect based on the information any need for a human resource, and to generate the one or more request signals for the control unit based on any such detection. Thus the request signals may be generated by the control unit itself. In some examples, the detection may be based on use of one or more digital twins 54 for the patients. The module may, by way of example, be communicatively coupled with the one or more patient digital twin models 54 (where such models are included), and may monitor parameters of the patient models for detecting any need for a human resource by any of the patients.

In some examples, the digital models 54 of the patients themselves may be configured with functionality for generating and outputting the resource request signals responsive to a modelled physical state of the patient reflected by the model exhibiting a pathology or problem which calls for intervention or assistance by a human operator.

Example implementation of the control unit 10 in use will now be described.

In general, where a system, for example a hospital or factory, is running efficiently, and requirements for human intervention are being met as they arise by human operators, there may be no need for action by the control unit, and no need to collect sensor data for the human operators. Thus, the sensors for the human operators can by default be kept deactivated (e.g. powered down, or in a standby or idle mode).

If an unmet need for human intervention arises, e.g. as indicated by a digital twin of a patient in a hospital, at this point the control unit 10 may take action, and respond by activating sensors for a relevant subset of the human operators, and collecting data, to use in determining the optimal one or more operators to respond to the need for human intervention. Sensors are thus switched on, data collection starts, and the most appropriate human resource is directed to carry out the required action. The most appropriate human resource is in general not only the person who is available and at a nearest location, but may also be one who is most rested, concentrated, focused and knowledgeable in the clinical area relevant to the human resource need. In other words, a human operator may be selected who is most fitted to the task.

After collection of the relevant human operator sensor data for making this determination, the sensors may be deactivated again (e.g. powered down or put into idle or standby mode). This may be done after passage of a default period of time, for example after a defined period of time of inactivity of the sensors (i.e. when data is no longer being collected). This may be done by the control unit, or the sensors themselves may be configured with an auto-shutdown or auto-idle function, having the effect of powering down or putting the sensor into standby or idle mode after a certain period of inactivity.

An example operation of the control unit in use may run as follows.

First an event occurs in relation to a patient or machine in the hospital or factory which requires human intervention.

This event, and the consequent need for human intervention is detected, for example by a digital twin of the patient or machine. Based on need for a human resource (human intervention), a signal is issued to the control unit (or to a processing component of the control unit) indicative of the need for human intervention. Based on this, it is determined which of the multiple types of human operator are appropriate types or categories for responding to the need for human intervention, e.g. nurses and doctors. Following this, for all human operators 22 within the system of those particular appropriate types, the sensor sets 24 carried by those operators are activated (e.g. powered up) and data is collected from the sensors.

The digital twins 52 for each of the human operators 22 are updated using the sensor data collected for the respective human operators.

The sensors 24 of the human operators may then be powered off.

Following this, the workforce scheduling algorithm 56 (or a different component) determines, based on output parameters of the digital models 52 of the human operators an optimal selection of one or more human operators for responding to the need for human intervention.

The proposed selection is communicated to the user interface 60 for review by a manager or shift leader (a responsible person). The user may confirm or adjust the selection using the user interface 60 and this decision is submitted to the control unit 10 from the user interface. In case the user is not available to review the selection, the selection proposed by the control unit may automatically be implemented. For example there may be an auto- time out feature whereby after a pre-defined time period, the selection is automatically implemented without confirmation at the UI.

The selection decision is communicated to each of the selected one or more human operators 22, for example via a mobile communication device carried by each human operator. Optionally, each operator may have the option to reject their own selection, for example if they are already engaged in an urgent action, or if they do not have the sufficient skill or knowledge to respond to the incident they have been assigned to.

Each human operator who accepts the assignment then responds to the request for intervention by attending the patient or machine in need of assistance.

Examples in accordance with a further aspect of the invention provide a control method for interacting with a system of resources.

The system for example comprises: a plurality of human operators, each of a particular human operator type, and each human operator carrying on their person a set of one or more sensors for use in monitoring at least one of: location of the operator and a physiological parameter of the operator.

The method comprises:
receiving request signals indicative of a need for a particular human resource, the human resource associated with a particular one or more of the types of human operator; and
controlling activation and deactivation of the sensors sets of the plurality of human operators dependent on received request signals.

One example method 80 in accordance with one or more embodiments is outlined in block diagram form in Fig. 2.

The method comprises receiving 84 request signals indicative of a need for a particular human resource.

The method further includes a step of associating 86 the human resource need indicated by the request signal(s) with one or more particular human operator types, e.g. doctor, nurse, medical imaging operator, anesthetist, physiotherapist etc. This step thus comprises determining which types of human operator are associated with the human resource need, i.e. which types or categories are capable of, or appropriate for, responding to the need. For example, the human resource need may be a requirement for a blood test for the patient to check red and white cell count. Any of a doctor, nurse, or phlebotomist are capable of taking the blood from the patient. Hence, the types of human operator associated with this need for human resource in this case include doctors, nurses, and phlebotomists, as these are capable of responding to the need for intervention.

The method further includes controlling 88 activation of the sensors sets of the plurality of human operators associated with the human resource need. Data may then be collected from the sensor sets.

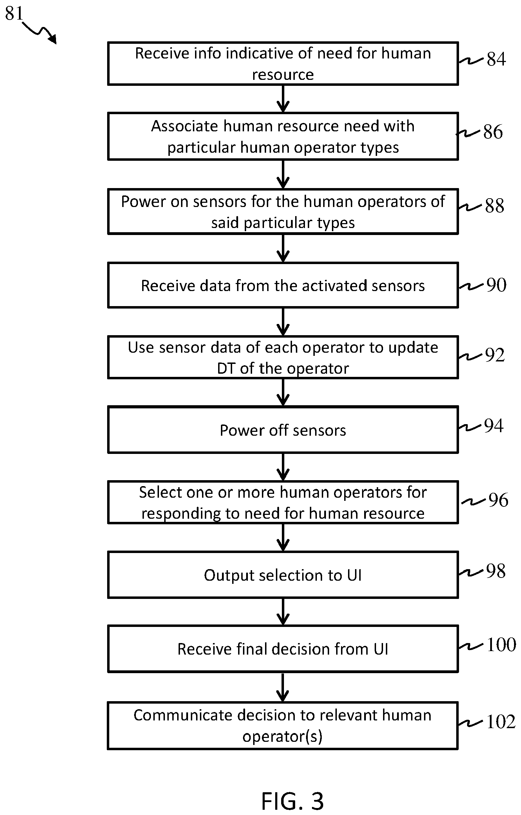

Fig. 3 shows a further example method 81 in accordance with one or more embodiments, which includes advantageous additional method steps performed subsequent to the steps of Fig. 2.

For this method, the control unit 10 is further assumed as including a data storage unit, the data storage unit storing a personalized digital model for each of the human operators configured to model for the operator one or more parameters, which may include by way of example at least one or more of: location, agenda, stress level, time on duty since a rest break, fatigue level, clinical experience.

These of course merely represent an exemplary subset of possible parameters which a digital twin of a human operator may model. A wide variety of different parameters may be modelled for the human operator to assist in the selection of an optimum one or more operators for a particular call. Further modelled parameters might include cumulative physical movement of the user (i.e. indicative of physical exertion); computer usage data (e.g. text generated by the user during their shift, which may be indicative of mental exertion), speech data (e.g. duration/energy of speech, e.g. measured by one or more acoustic transducers), and/or information about previous cases attended to during the same shift (e.g. severity of cases, number of patients).

Each modelled parameter may be determined or calculated based on input sensor data and one or more algorithms for instance.

It will be known to the skilled person approaches for quantifying or estimating the above factors, such as stress level, fatigue level and alertness, based on physiological parameters for instance. For example, SpO2 and heart rate are sometimes used to estimate stress level. By way of further example, fatigue has been known to be estimated based on a combination of sensor data indicative of one or more of: electrical activity of heart, brain, muscles and skin potentials as well as temperature, position/posture, and respiration. These are mentioned merely by way of illustration.

The method comprises receiving 84 request signals indicative of a need for a particular human resource.

As in the method of Fig. 2, the method further includes a step of associating 86 the human resource need indicated by the request signal(s) with one or more particular human operator types.

The method further comprises controlling activation 88 of the sensors sets (powering on the sensor sets) of the plurality of human operators associated with the human resource need.

Sensor data is then received 90 from the powered on sensors.

As discussed above, the sensor data is then used to update 92 the personal digital models (digital twins) of each of the human operators whose sensors were activated. As discussed above, the digital models of the human operators may include for each operator an activity record, agenda, clinical role, knowledge base, current location, and also fatigue, stress level, hours since a break and so on. The sensors can include at least a location sensor and one or more physiological parameter sensors. The location sensor can be used to update in the personal digital model the location of the operator, and also their record of activity over for instance their shift. The physiological parameter sensors (e.g. heart rate, blood pressure, blood oxygen level), may be used to update input parameters of the digital model, and the digital model may use these to determine model output parameters including stress level, and/or fatigue of the operator.

Once the digital twins of the human operator are updated, the sensors of the human operators are deactivated 94 again (e.g. powered off or down, or put into idle or standby mode).

Following this, the method 81 comprises selecting 96 an optimum one or more of the human operators for attending or responding to the need for human resource indicated by the request signal, based on the updated digital models of the human operators. This may be for instance based on positional proximity of each of the human operators to the patient or machine which requires human intervention in combination with fatigue level, clinical expertise in the relevant area, upcoming appointments of the operator and other factors.

Once the selection of an optimum one or more operators has been made, the method 81 preferably further comprises generating a data output 98 representative of the selection for transmitting to a user interface (UI) device. The UI may include a display for displaying the selection result to a user. The UI may include a user input for receiving control commands from a user, for example indicative of approval or non-approval of the proposed selection of human operators. The user interface may permit a user to amend the suggested selection of one or more human operators. For instance the user may be a manager or shift leader, and can use their knowledge and experience to check that the proposed human operators are the most appropriate for responding to the need for intervention.

The final selection of human operators is thus preferably made using the UI, either by the user confirming the selection proposed by the control unit, or by amending the selection and submitting it to the control unit. The control unit receives 100 this final selection from the UI.

This final selection decision may then be communicated 102 to the one or more selected human operators. For example, a message may be transmitted to each of the selected human operators via a personal mobile communication device carried by each operator.

Optionally, there may include the facility for each operator to either accept or reject the call for assistance, for example if they are already attending to an urgent situation which they cannot leave. If an operator rejects the call, the next best operator may be selected, and a call message sent to them instead for example.

Although in the above example, deactivation 94 of the sensor sets of the operators is done immediately after collecting data from the sensors and updating the human operator digital twins, this is not essential. In further embodiments, deactivation of the sensors can be done after completion of one or more further steps or processes. For example, it may be done as the last step of the method 81 of Fig. 3, for example after the final decision regarding the human operator assignments has been made 100 or after it has been communicated 102 to the human operators.

This approach carries the advantage that the selection of human operators can be more readily adjusted in the event that the operator viewing the UI or one or more of the selected operators reject the proposed selection of operators, e.g. because there is a scheduling clash. If this occurs, the sensors are still active, permitting more data to be potentially collected from the operator sensors sets and for the selection of the optimum one or more operators to be made again. This can be done without having to reactivate the sensors and await initialization of the sensors, meaning a new selection can be determined more quickly.

A number of example applications of control devices and method according to embodiments of the invention for particular use cases will now be described.

One example application of embodiments of the system and method of the invention is for use within an intensive care unit (ICU) of a hospital.

Managing an ICU requires many ad hoc decisions a day by shift leaders, charge nurses and doctors, and requires information to be available accurately and promptly. The number, types and conditions of patients continuously varies, and consequently also the required human resources (for example the number of required human operators, clinical specialty, clinical knowledge).

One example application of embodiments of the present invention might occur for instance in the event that a patient deteriorates, for example by showing new-onset arterial fibrillation, and thus requires help. The system 20 or control unit 10 may include digital twins for each of the patients in the ICU which model the current physical state of each respective patient based on patient data (as discussed above).

The new-onset arterial fibrillation may be detected by the patient's digital twin. It may for example detect a change in the physical state of the patient, may perform a clinical interpretation of this change (e.g. with a triaging algorithm) and thus determine the arterial fibrillation, and so a need for human intervention by a clinician. Based on the pathology detected by the digital twin, a determination is made as to what human resources are required. This may be done by the digital twin or by another processing component or module of the control unit. For example, it may be determined that the patient requires a lab test, and an in-person consultation of a cardiologist and a nurse as soon as possible.

A request signal indicative of the need for these human resources is generated.

Responsive to this request signal, the control unit first determines the particular categories of human operator appropriate for providing these human resources. In this case, these consist of all cardiologists, nurses and lab personnel.

Based on this, the control unit 10 issues a control command for triggering activation of the sensor sets being carried by the population of nurses, cardiologists and lab personnel. In particular, the sensors carried by these human operators are switched on (powered up), sensor readings collected, and the required data and information transmitted to and received at the control unit. The sensor data from each of the sets of sensors is fed to relevant human operator digital twin, for updating or developing the digital twin so as to reflect a current real-time state of the human operator.

After the digital twins for the human operators have been updated, the sensors are de-activated.

Following this, a workforce-scheduling algorithm selects and plans a determined best (and optionally also second best) combination of cardiologist, nurse and medical laboratory assistant to carry out the required activities. This determination may be based on use of the updated digital models for the human operators. For example, output parameters of the models may be probed or examined and used to make comparisons between different of the operators to determine a best one or more for responding to the need for intervention. For example, position of the operators may be taken into account, as well as optionally their stress or fatigue level, their upcoming appointments, their experience level in the area concerned, and any other relevant factors.

The best scenario may be decided on the basis of different possible criteria. Possible criteria for assessment might include the speed with which a resource request can be responded to (e.g. closer operators are able to attend sooner, operators with no current appointments can attend quicker), and/or efficiency of human resource allocation (for example, to conserve high-skill staff, for each task the lowest skilled operator able to deal with the task may be assigned (e.g. a nurse rather than a doctor)).

Hence, following a speed criterion, an optimization may be performed between proximity of operators, and the level of rest and knowledge of operators. For a resource allocation efficiency criterion, a priority may be given to optimizing the distribution of workload among operators in order to secure the availability and quality of human resources in the system of resources over time.

Further criterion for selecting the optimum one or more human operators may include the minimizing of certain pre-defined system costs, such as minimizing disruption of already running workflows (e.g. not cancelling already booked appointments), or minimizing gaps in shift schedules. In case the selected best one or more operators is not possible, for example because one human operator rejects the call for action, a second best selection may instead be deployed, or if necessary a third selection, and so forth.

The selection may be output to a user interface device for reviewing by a user, for example a manager or shift leader, e.g. the charge nurse. For example, an App installed on the control unit or elsewhere may communicate the scenario options (first and second best selection) to for instance a dashboard used by the charge nurse, who may then make a final decision on the human operators to be assigned to the task. Once this decision is submitted, the control unit may update the work schedule of each of the selected human operators. The selected human operators (lab, cardiologist, nurse) may receive a notification of an update in their work schedule.

In some instances, recent sensor data may already be available for a particular human operator, for example from a recent activation of the sensors responsive to another resource request. In this case, in some examples the control unit may detect the recent data, and only activate sensors for the human operator that add to, or augment, the recent data that has been collected. In some examples, a pre-defined time threshold may be set for the maximum age of recently collected data before new data is required to be collected. The control unit may be configured to check the age of any previously collected sensor data for each human operator, and if it exceeds the age threshold, may activate the relevant sensor(s) of the human operator to collect new data.

In some examples, the control unit may activate only a subset of the set of sensors carried by each human operator, for example dependent on the particular human resource need indicated by the request signal. For example, for certain intervention needs, only certain information may be relevant. For example, for a very simple task, requiring minimal skill, only information on the availability of a human operator is needed. By contrast, for a more complex need, such as a surgical intervention, also information on the physical and mental state of the human operator (e.g. surgeon) may be relevant. Thus, different subsets of the sensors of each of the particular types of human operators may be activated depending upon the human resource requirement.

A further example application of embodiments of the device and method of the invention is for use within a factory or plant. In this case, instead of patients, the resource-consuming entities of the system may include robots, or conveyor-belts for instance.

In "Industry 4.0" style factories, people will still be essential and will work collaboratively with robots. For this example application, examples of human resource requirements may include a sudden, unexpected need for repair of a machine (for instance, as indicated by a digital twin of a particular machine), or a sudden supply chain issue that needs attending to. By way of example, each machine, robot, or system in the factory may have a digital twin associated with it for modelling a live state of the machine or system, and for detecting or predicting faults with the machine or system in real time. These can be used to generate requests for human resources, i.e. intervention by a human operator.

Responsive to a detected need for a human resource (e.g. to a resource request signal) the control unit determines a relevant subset of categories of human operator type (e.g. machine repair technicians, mechanics, engineers, programmers, systems experts etc.), and triggers activation of the sensor sets carried by the relevant categories of human operator. The sensors of these operators are thus switched on and data readings read from them.

The sensor data is transmitted to the control unit and the digital twins for the respective human operators are updated. After this, the sensors are de-activated. Subsequently, a workforce scheduling algorithm selects and plans the best operator(s) to deploy for responding to the need for human intervention.

There will now be outlined a number of optional features and modifications which may be applied to any embodiment of a control unit or control method outlined in this disclosure, or outlined in any claim of this application.

According to one or more embodiments, the activation of the sensors of said particular one or more types of human operators associated with the human resource requirement may be done in stepwise or piecemeal or iterative fashion. For example, a first sensor of each of the sensor sets may first be activated, and data retrieved and fed to the control unit. If the data from these first sensors is not sufficient to narrow down one (or a pre-defined number of) optimum human operator(s), then a next sensor may be activated for each operator, and data collected, and so on, in stepwise fashion until one (or more) human operators can be selected as optimum.

For example, there may first be activated the location sensors for each human operator to identify the operators who are closest to the resource requirement. If multiple operators are at the same distance, then further sensors may be activated and data collected (e.g. heart rate sensors, and assessment made of stress or fatigue level of each of the equidistant human operators).

In accordance with one or more embodiments, the control unit may have a learning function allowing it to learn past patterns of sensor activation and to use this data to inform the activation and deactivation of sensors responsive to each new resource request. For example, if the past data indicates that resource requests have tended to occur with a greater frequency during a particular time frame (a particular time frame of the day), then the control unit may be configured, if within that time frame, to keep sensors active after responding to a resource request rather than deactivating them (i.e. because it is likely that a new call will be soon received).

In accordance with one or more embodiments, the control unit or method may make use of one or more machine learning algorithms. For example, the control unit may employ a machine learning algorithm configured for improving future selection of the one or more human operators for responding to human resource requests based on prior selections and their outcomes. For example, for each run of the method steps 81 of Fig. 3, a machine learning algorithm may receive, as training data, data entries indicative of the collected human operator sensor data, and the final selection of human operator(s) made by the control unit, and wherein each of these is labelled or tagged with the outcome for the patient or machine (i.e. improvement or non-improvement of the medical or other situation requiring intervention), and possibly other key performance indicators such as response time and/or cost. The machine learning algorithm may be configured to continuously improve the performance of the control unit in performing the selection of human operators.

The control unit and the cloud-based processing unit can be implemented in numerous ways, with software and/or hardware, to perform the various functions required. A processor is one example of such unit which employs one or more microprocessors that may be programmed using software (e.g., microcode) to perform the required functions. Such unit may however be implemented with or without employing a processor, and also may be implemented as a combination of dedicated hardware to perform some functions and a processor (e.g., one or more programmed microprocessors and associated circuitry) to perform other functions. Examples of components that may be employed in various embodiments of the present disclosure include, but are not limited to, conventional microprocessors, application specific integrated circuits (ASICs), and field-programmable gate arrays (FPGAs).

In various implementations, a processor or control unit may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a control unit or processor or may be transportable, such that the one or more programs stored thereon can be loaded into a processor or control unit.

The skilled person would be readily capable of developing a control unit for carrying out any herein described method. Thus, each step of a flow chart may represent a different action performed by a control unit or processor, and may be performed by a respective module of the control unit or processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A control unit (10) for interacting with a plurality of human operators (22), each of a particular human operator type, and each human operator carrying a sensor set (24) of one or more sensors for use in monitoring at least one of: location of the operator and a physiological parameter of the operator,
the control unit (10) configured for receiving request signals indicative of a need for a particular human resource, the human resource associated with a particular one or more of the types of human operator; to control activation and deactivation of the sensors sets (24) of the plurality of human operators (22) dependent on received request signals.

2. A control unit (10) as claimed in claim 1, wherein the control unit is configured, responsive to each received request signal, to trigger activation of the sensor sets (24) for each of the plurality of human operators of said particular one or more types.

3. A control unit (10) as claimed in claim 1 or 2,
wherein the control unit (10) further comprises a data storage unit for storing a personalized digital model (52) for each of the human operators (22) configured to model for the operator at least one or more of: location, agenda, stress level, time on duty since a rest break, fatigue level, clinical experience, and
wherein said sensor sets (24) are for collecting data suitable for updating said personalized digital models (52).

4. A control unit (10) as claimed in claim 3, wherein the control unit is configured, subsequent to activation of said sensor sets (24) of said particular types of human operator, to collect data from each of the sensor sets, and to update each digital model (52) of each human operator of said particular one or more types based on the data collected from the relevant operator's sensor set.

5. A control unit (10) as claimed in claim 4, the control unit further configured to trigger deactivation of said sensor sets (24) of the particular one or more types of human operators (22) subsequent to updating the digital models (52).

6. A control unit (10) as claimed in claim 4 or 5, wherein the control unit is further configured to select one or more of the human operators (22) of said particular one or more types based on data from the updated personal digital models (52) of said human operators.

7. A control unit (10) as claimed in any of claims 1-6, wherein the control unit is configured, responsive to receipt of the request signal, to perform a step of determining the particular types of human operator (22) associated with the human resource indicated by the request signal.

8. A control unit (10) as claimed in any of claims 1-7, wherein the control unit includes a resource request module arranged to recurrently or continuously receive information indicative of a current state of one or more patients (26), to detect based on the information any need for a human resource, and to generate the one or more request signals for the control unit based on any such detection.

9. A control unit (10) as claimed in claim 8, wherein the detecting the need for a human resource is based on use of one or more personal digital models (54) of the one or more patients (26).

10. A control method (80, 81) for interacting with a plurality of human operators (22), each of a particular human operator type, and each human operator carrying a sensor set (24) of one or more sensors for use in monitoring at least one of: location of the operator and a physiological parameter of the human operator,
the method comprising
receiving (84) request signals indicative of a need for a particular human resource, the human resource associated with a particular one or more of the types of human operator; and
controlling (88) activation and deactivation of the sensors sets (24) of the plurality of human operators (22) dependent on received request signals.

11. A method (80, 81) as claimed in claim 10, further comprising, responsive to receipt of the request signal, performing a step of determining the particular types of human operator associated with the human resource indicated by the request signal.

12. A method (80, 81) as claimed in claim 10 or 11, wherein
the system further comprises data storage unit, the data storage unit storing a personalized digital model (52) for each of the human operators (22) configured to model for the operator at least one or more of: location, agenda, fatigue level, stress level, time on duty since a rest break, and clinical experience; and
the method further comprising, subsequent to activation of said sensor sets (24) of said particular types of human operator, collecting data from each of the sensor sets, and updating each digital model (52) of each human operator (22) of said particular one or more types based on the data collected from said operator's sensor set (24).

13. A method (80, 81) as claimed in claim 12, further comprising triggering deactivation of said sensor sets (24) of the particular one or more types of human operators (22) subsequent to updating the digital models (52) of the human operators.

14. A method (80, 81) as claimed in claims 12 or 13, further comprising selecting one or more of the human operators (22) of said particular one or more types based on data from the updated personal digital models (52) of said human operators.

15. A computer program product comprising code unit configured, when run on a processor, the processor being communicatively coupled with sensor sets (54) carried by a plurality of human operators (22), to cause the processor to perform the method of any of claims 10-14.

16. A human resource management system, comprising a control unit (10) as claimed in any of claims 1-9, and a plurality of sensor sets (24) of one or more sensors for use in monitoring at least one of: location of a human operator (22) and a physiological parameter of the human operator.
